Europäisches Patentamt

European Patent Office

Office européen des brevets

(11) Veröffentlichungsnummer: **0 174 012**
**B1**

(12) **EUROPÄISCHE PATENTSCHRIFT**

(45) Veröffentlichungstag der Patentschrift:
**29.11.89**

(21) Anmeldenummer: **85111139.3**

(22) Anmeldetag: **04.09.85**

(51) Int. Cl.⁴: **G01N 33/12, G01N 21/59**

(54) Vorrichtung zum Durchstrahlen von biologischem Gewebe mit Licht.

(30) Priorität: **07.09.84 DE 3432955**

(43) Veröffentlichungstag der Anmeldung:
**12.03.86 Patentblatt 86/11**

(45) Bekanntmachung des Hinweises auf die Patenterteilung:
**29.11.89 Patentblatt 89/48**

(84) Benannte Vertragsstaaten:
**AT BE CH DE FR GB IT LI LU NL SE**

(56) Entgegenhaltungen:
**AT-B- 370 884**
**DE-A- 2 422 905**
**DE-C- 632 641**

(73) Patentinhaber: **Reyer, Michael, Bussestrasse 6,
D-2850 Bremerhaven(DE)**

(72) Erfinder: **Reyer, Michael, Bussestrasse 6,
D-2850 Bremerhaven(DE)**

(74) Vertreter: **Patentanwälte Dipl.-Ing. F.W. Möll Dipl.-Ing.
H.Ch. Bitterich, Langstrasse 5 Postfach 2080,
D-6740 Landau/Pfalz(DE)**

## Beschreibung

Die Erfindung betrifft eine Vorrichtung zum Durchstrahlen von biologischem Gewebe mit Licht zur Sichtbarmachung von darin enthaltenen Körpern, enthaltend eine lichtdurchlässige Arbeitsplatte und wenigstens eine künstliche Lichtquelle, die unter der Arbeitsplatte angeordnet ist und deren Licht über ein optisches System gebündelt auf die Arbeitsplatte gerichtet wird.

Eine derartige Durchleuchtungsvorrichtung für Fischfleisch ist bekannt aus der DE-PS 632 641. Es handelt sich dabei um ein zum Einsetzen in die Aussparung eines Arbeitstisches geeignetes Rahmengestell, in dessen Oberseite eine Glasplatte eingesetzt ist. Die Glasplatte dient als Arbeitsplatte, auf der die Fische ausgenommen, filetiert und versandfertig gemacht werden. Unter der Glasplatte befindet sich eine Lichtquelle, deren Lichtschein auf die Glasplatte fällt, wobei mit Hilfe von Spiegeln eine Verstärkung und Vergleichmäßigung der Leuchtstärke auf der Arbeitsplatte erzielt wird.

Mit diesem bekannten Gerät konnten bereits 3 bis 4 cm lange, streichholzdicke Würmer auch in bis zu 8 cm dicken Fischfilet erkannt werden. Das Arbeiten an dem Gerät ist jedoch nur mit Spezialbrillen möglich, da die relativ große, hell beleuchtete Arbeitsfläche leicht zu Augenschädigungen führt.

Neben dem Befall mit Würmern hat bei Fischen auch der Befall mit sonstigen Krankheiten stark zugenommen. Es handelt sich dabei beispielsweise um Verknorpelungen oder Nekrosen, die im wesentlichen durch in die Meere eingeleitete Schadstoffe ausgelöst werden. Zwar sollen nach der herrschenden Meinung weder die Würmer, noch die sonstigen Veränderungen des Fischfleisches gesundheitsschädlich sein, trotzdem stellen sie eine wesentliche Beeinträchtigung des Lebensmittels Fisch dar und wirken durchaus ekelerregend. Soll der Absatz von Seefisch unverändert bleiben, so müssen diese befallenen Stellen ebenso wie die Parasiten sorgfältig beseitigt werden, bevor das Fischfleisch in den Handel gelangt.

Ein weiteres Anwendungsgebiet der Durchleuchtung ist beispielsweise der menschliche Körper. Hierzu werden seit Jahrzehnten fast ausschließlich Röntgenstrahlen verwendet; in neuerer Zeit sind auch andere harte Strahlen, zum Beispiel Neutronenstrahlen usw., eingesetzt worden. Die mit der Verwendung von harten Strahlen verbundene Gefahr für das lebende Gewebe ist bekannt.

Es wird deshalb versucht, in der Medizin die Verwendung von harten Strahlen zu reduzieren, und stattdessen andere Mittel einzusetzen, um das normalerweise unzugängliche Innere des menschlichen Körpers sichtbar zu machen. Als Beispiel sei erwähnt die Verwendung von Ultraschallwellen.

Es liegt eigentlich nahe, auch das sichtbare Licht zur Durchleuchtung des menschlichen Körpers bzw. allgemein von biologischem Gewebe einzusetzen. Durchschlagende Erfolge sind jedoch bisher nicht bekannt geworden. Eine gewisse Anwendung findet das sichtbare Licht bei den bekannten elektronischen Pulszählern, die beispielsweise auf das Ohrläppchen aufgesteckt werden und die die sich mit dem Pulsschlag bzw. mit dem Blutfluß verändernde Transparenz des Ohrläppchens auswerten. Eine Durchleuchtung des Gewebes im Sinne des Aufspürens von Fremdkörpern usw. wie es heute allgemein mit Hilfe von Röntgenstrahlen geschieht, findet dabei jedoch nicht statt.

Der vorliegenden Erfindung liegt deshalb die Aufgabe zugrunde, die eingangs beschriebene Durchleuchtungsvorrichtung derart weiterzubilden, daß damit auch lebendiges biologisches Gewebe beliebiger Art durchstrahlt werden kann, um die innere Struktur – Organe, Blutgefäße, Knochen, Knochenbrüche, Fremdkörper, Veränderungen des Gewebes, sichtbar zu machen, ohne daß dabei eine Schädigung des untersuchten Gewebes oder der untersuchenden Person befürchtet werden muß.

Diese Aufgabe wird dadurch gelöst, daß das Licht so gebündelt ist, daß der virtuelle Brennpunkt in einem Abstand oberhalb der Oberfläche der Arbeitsplatte liegt, daß in einem Abstand unter der Oberfläche der Arbeitsplatte eine lichtstreuende Fläche angeordnet ist und daß die Lichtquelle so angeordnet und abgeschirmt ist, daß kein direktes Licht die Arbeitsplatte trifft.

Der wesentliche Vorteil dieser Vorrichtung liegt darin, daß eine schädigende Wirkung von sichtbarem Licht auf biologisches, insbesondere menschliches Gewebe unbekannt ist, so daß auch eine langdauernde Durchleuchtung, beispielsweise bei Operationen, Untersuchungen mit Sonden usw. möglich ist. Insbesondere hat sich gezeigt, daß durch die Verwendung von gebündeltem Licht, welches an einer lichtstreuenden Schicht knapp unterhalb der Arbeitsfläche gestreut wird, das Innere des durchstrahlten Gewebes einwandfrei erkennbar wird.

Die hervorragende Abbildung beruht nicht auf einer besonders großen Beleuchtungsstärke; hiervon kann man sich leicht überzeugen, wenn man beispielsweise die Hand direkt ins sehr helle Sonnenlicht hält. Die hervorragende Abbildung beruht vielmehr auf der speziellen Lichtführung. Aus diesem Grunde soll auch verhindert werden, daß direktes Licht von der Lichtquelle auf die Arbeitsplatte fällt.

Wesentliche Vorteile der erfindungsgemäßen Lösung bestehen in dem Fehlen jeglicher Beschädigung des biologischen Gewebes, in einer Beschleunigung des Untersuchungsvorganges sowie in einer erheblich verbesserten Augenschonung.

Die Augenschonung ist besonders groß, wenn der beleuchtete Teil der Arbeitsplatte entweder nur als schmaler Streifen ausgebildet ist, der unter dem zu untersuchenden Gewebe hin- und hergeschoben werden kann, oder aber, wenn mit Hilfe von einstellbaren Blenden der beleuchtete Teil der Arbeitsplatte nur auf den zu untersuchenden Bereich begrenzt wird.

Außerdem hat es sich gezeigt, daß eine Augenschonung auch dadurch erreicht wird, daß am Rande der gleichmäßig und hell beleuchteten Fläche ein Streifen verminderter Helligkeit erzeugt wird, so daß der Hell-Dunkel-Übergang abgemildert wird.

Um die bei einer Durchleuchtung von insbesondere lebendem biologischem Gewebe störende Infrarotstrahlung der Lichtquelle reduzieren zu können, empfiehlt es sich, im Lichtweg infrarotdurchlässige Spiegel oder auch Infrarotsperrfilter anzuordnen. Außerdem ist es möglich, die Arbeitsplatte selbst forciert zu kühlen. Dabei sind alle Kühlungsarten von der Luftkühlung bis zur Wärmepumpe grundsätzlich geeignet.

Während bei einer Anwendung der Durchleuchtungsvorrichtung in der Medizin die beleuchtete Fläche der Arbeitsplatte mit Hilfe von Blenden auf die zu untersuchende Stelle eingegrenzt werden kann, kann dieses Prinzip bei der Anwendung der Durchleuchtungsvorrichtung in einem Produktionsbetrieb, beispielsweise bei der Durchleuchtung und Säuberung von Fischfilet, nicht beibehalten werden. In einem solchen Fall wird nur eine schmale, streifenförmige Fläche der Arbeitsplatte beleuchtet, um eine optimale Augenschonung zu erreichen, und gleichzeitig die Lichtfläche unter der Arbeitsplatte hin- und herbewegt, so daß nacheinander alle zu untersuchenden Teile durchleuchtet werden. Da hierdurch der restliche Teil der Arbeitsplatte dunkel bleibt, ergibt sich eine gegenüber der eingangs beschriebenen bekannten Durchleuchtungsvorrichtung erheblich verbesserte Schonung der Augen des Personals, so daß auf die Verwendung von Spezialbrillen verzichtet werden kann. Außerdem ermöglicht die stärkere Bündelung des Lichts entweder eine Erhöhung der Helligkeit oder aber eine Reduzierung der Lampenleistung. Gleichzeitig wird die von der unerwünschten Wärmestrahlung ausgelöste Erwärmung des Fischfleisches ebenfalls auf den jeweils beleuchteten schmalen Streifen beschränkt.

Vorzugsweise ist die Lichtquelle in einen Lichtkasten eingesetzt, wobei der Lichtkasten an Führungsschienen leichtgängig gelagert ist. Der Lichtkasten selbst wird vorzugsweise mit Endloskette und Elektromotor bewegt.

Um zu verhindern, daß beim Hantieren mit gekühlten Fischfilets auf der Unterseite der durch den Lichtstrahl erwärmten Arbeitsplatte sich eine Schicht von Kondenswasser bildet, sind an dem Lichtkasten vorzugsweise je ein Abstreifer rechts und links der Lichtquelle angeordnet, die die Unterseite der Arbeitsplatte ständig säubern.

Um zu verhindern, daß die lichtstreuende Schicht, die für die Durchleuchtungswirkung entscheidend ist und gemäß einer bevorzugten Ausbildung aus Diamantstaub besteht, durch Schmutz, Feuchtigkeit usw. unwirksam gemacht wird, ist diese lichtstreuende Schicht vorteilhafterweise zwischen zwei Glasplatten eingeschlossen, von denen die obere vorzugsweise die Arbeitsplatte selbst darstellt.

Es hat sich gezeigt, daß bereits relativ geringe Unterschiede in der Dicke der Arbeitsplatte und daraus resultierend Unterschiede im Abstand zwischen lichtstreuender Schicht und biologischem Gewebe die Durchleuchtungswirkung verschlechtern können. Mit Hilfe einer Justierung des Abstandes zwischen optischem System und der Oberfläche der Arbeitsplatte kann dies jedoch ausgeglichen werden.

Anhand der Zeichnung soll die Erfindung in Form eines Ausführungsbeispiels näher erläutert werden. Es zeigen

Fig. 1 einen Querschnitt durch eine erste Ausführungsform einer Lichtquelle,

Fig. 2 einen Querschnitt durch eine Durchleuchtungsvorrichtung mit beweglicher Lichtquelle,

Fig. 3 einen Längsschnitt durch den Lichtkasten der Fig. 2 und

Fig. 4 einen Querschnitt durch eine weitere Ausführungsform einer Durchleuchtungsvorrichtung.

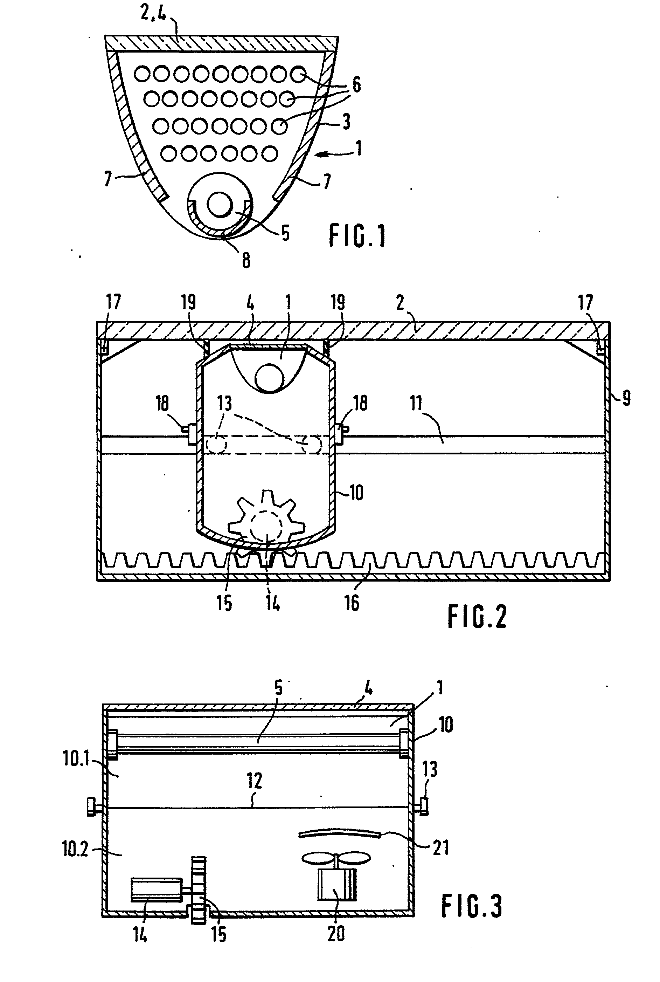

In Fig. 1 erkennt man eine Lichtquelle 1, bestehend aus einem länglichen Gehäuse 3 mit etwa parabolischem Querschnitt. Das Gehäuse 3 ist an den Seitenwänden mit Spiegeln 7 belegt. Die Oberseite des Gehäuses 3 ist mit einer Glasplatte 4 geschlossen. Dient die Glasplatte 4 gleichzeitig als Arbeitsplatte 2, so ist sie an ihrer Unterseite mattiert, um eine schattenfreie Ausleuchtung zu gewährleisten. Diese Lichtquelle 1 kann in ein geeignetes Gehäuse eingesetzt werden.

Im Brennpunkt des teilweise verspiegelten Gehäuses 3 erkennt man eine Röhrenleuchte 5, die ebenfalls einen Spiegel 8 besitzt. Die Spiegel 7, 8 lassen unerwünschte Infrarotstrahlung vorzugsweise ohne Reflektion passieren. Öffnungen 6 im Gehäuse 3 dienen der Abfuhr der in der Lampe 5 erzeugten Hitze mit Hilfe eines von einem Ventilator erzeugten Luftstroms. Entsprechende Konstruktionen sind von den Beleuchtungseinrichtungen für Operationstische bekannt.

Eine noch stärkere Kühlung kann erreicht werden, wenn man ein besonderes Kühlmittel, beispielsweise flüssiges Kohlendioxyd, direkt auf die als Arbeitsplatte 2 dienende Glasplatte 4 einwirken läßt. Die Platte 4 muß nicht unbedingt aus Glas bestehen, der Ausdruck Glas steht vielmehr stellvertretend für alle Materialien, die für den jeweiligen Anwendungszweck der Durchleuchtungsvorrichtung geeignet sind.

In Fig. 2 erkennt man einen Querschnitt durch ein etwa quaderförmiges Gehäuse 9, dessen offene Oberseite mit einer als Arbeitsplatte 2 dienenden Glasscheibe abgedeckt ist. Unterhalb der Arbeitsplatte 2 sind am Gehäuse 9 Ein-Aus- Schalter 17 angeordnet, mit deren Hilfe die Durchleuchtungsvorrichtung ein- und ausgeschaltet werden kann, beispielsweise durch einen Handdruck der Bedienungsperson.

Unterhalb der Arbeitsplatte 2 erkennt man die Lichtquelle 1. Sie befindet sich in einem speziellen Lichtkasten 10, der aus zwei Teilen 10.1, 10.2 besteht, die eine Trennlinie 12 (Fig. 3) aufweisen.

Links und rechts der Lichtquelle 1 sind Abstreifer 19, beispielsweise Gummilippen, angeordnet, die die Unterseite der Arbeitsplatte 2 von Schmutz und Feuchtigkeit reinigen. Dadurch ist das von der Lichtquelle 1 beleuchtete Teilstück der Arbeitsplatte 2 immer sauber, so daß die Lichtführung nicht behindert wird.

Der Lichtkasten 10 ist mit Hilfe von Kugellagern 13 in Führungsschienen 11 leichtgängig gelagert. Außen am Lichtkasten 10 erkennt man Endschalter 18.

Im Lichtkasten 10 erkennt man schließlich noch einen Antriebsmotor 14 mit einem Ritzel 15, welches mit einer im Gehäuse 9 gelagerten endlosen Kette 16 in Wirkverbindung steht und die Hin- und Herbewegung des Lichtkastens 10 erzeugt. Anstelle von Ritzel 15 und Zahnstange bzw. Kette 16 können auch andere Antriebe, beispielsweise Seilzüge, eingesetzt werden.

Fig. 3 schließlich zeigt den Lichtkasten 10 im Längsschnitt. Man erkennt an seiner Oberseite die Lichtquelle 1 mit der Röhrenleuchte 5 und die Glasplatte 4.

Im unteren Teil 10.2 des Lichtkastens 10 erkennt man das Ritzel 15, welches von dem wartungsfreien Elektromotor 14 angetrieben wird. Schließlich erkennt man noch den Ventilator 20 und ein Luftleitblech 21. Mit diesen Komponenten wird die Lichtquelle 1 indirekt gekühlt; außerdem können etwaige Feuchtigkeitsreste ausgetrocknet werden.

Bei der in Fig. 2 dargestellten Vorrichtung wird die Arbeitsplatte 2 nur noch in einem etwa 5 cm breiten, sich automatisch unter der Platte 2 hin- und herbewegenden Lichtband erleuchtet, wodurch die Augen der an der Beleuchtungsvorrichtung arbeitenden Bedienungsperson geschont werden. Das Tragen einer Schutzbrille ist nur noch bei überempfindlichen Augen erforderlich. Durch die Bündelung des Lichts ist die Durchdringung des biologischen Gewebes, beispielsweise des Fischfleisches, erheblich verbessert, wodurch ein effektiveres und leichteres Auffinden und Entfernen von Fremdkörpern, Parasiten usw. möglich ist. Die stufenlose Geschwindigkeitseinstellung von 0,5 bis 5 cm pro Sekunde ermöglicht die Anpassung an die Geschicklichkeit der Bedienperson, aber auch an die Stärke des Befalls durch Parasiten usw. Außerdem kann beim Umschalten der Bewegungsrichtung der Lichtquelle an beiden Enden der Führungsschiene eine Verzögerung zwischen 5 und 15 Sekunden gewählt werden, wodurch ein kontinuierliches Arbeiten, speziell beim Filetieren von Fischen, ermöglicht wird.

Das optische System 1 ist in einem im wesentlichen lichtdichten Gehäuse 40 angeordnet, wobei die Lichtquelle 5 in einem gesonderten Gehäuse angeordnet ist. Dadurch beispielsweise kann verhindert werden, daß Licht der Lichtquelle 5 direkt auf die Arbeitsplatte 29 gelangt.

Das Licht der Lichtquelle 5 wird mit Hilfe von Spiegeln 8, 25, 26 auf eine lichtbrechende Schicht 28 an der Unterseite der Arbeitsplatte 29 gebündelt, wobei der Brennpunkt bzw. die Brennlinie 31 des Lichtbündels im Abstand F oberhalb der Arbeitsplatte 29 liegt.

Die lichtbrechende Schicht 28, die optimale Ergebnisse bringt, wenn sie aus Diamantstaub besteht, ist zwischen zwei Glasplatten 29, 30 eingeschlossen, die zusammen die Arbeitsplatte bilden. Dadurch wird verhindert, daß durch Schmutz, Fingerabdrücke, Kondenswasser und dergleichen die optische Wirkung der lichtstreuenden Schicht 28 verschlechtert wird.

Um die Größe der beleuchteten Fläche 32 der Arbeitsplatte 29 verkleinern zu können, sind bewegliche Blenden 23, 24 vorgesehen. Diese Blenden können an verschiedenen Stellen im Lichtweg angebracht sein. Die Zeichnung zeigt Blenden 23 mit Abstand unterhalb der Arbeitsplatte 29 oder als Alternative Blenden 24 in unmittelbarer Nähe der Lichtquelle 5. Mit jeweils vier zusammengehörigen Blenden 23 bzw. 24 läßt sich die beleuchtete Fläche 32 der Arbeitsplatte 29 auf die jeweils benötigte Größe einstellen.

Es versteht sich von selbst, daß die beleuchtete Fläche 32 der Arbeitsplatte 29 gleichmäßig hell sein muß. Zur Augenschonung empfiehlt es sich, am Rand dieser gleichmäßig hell beleuchteten Fläche 32 einen beleuchteten Streifen verringerter Helligkeit zu erzeugen, um den Übergang zwischen beleuchteter und unbeleuchteter Fläche der Arbeitsplatte 29 fließend zu gestalten. Eine solche Schattenwirkung wird beispielsweise erreicht, wenn die Blenden 23 mit Abstand unterhalb der Arbeitsplatte 29 bzw. der lichtstreuenden Schicht 28 angeordnet sind.

Die Kühlung des optischen Systems ist durch einen Lüfter 20 repräsentiert. Es versteht sich von selbst, daß statt eines Lüfters auch intensivere Kühlsysteme, insbesondere Verdampfersysteme, eingesetzt werden können.

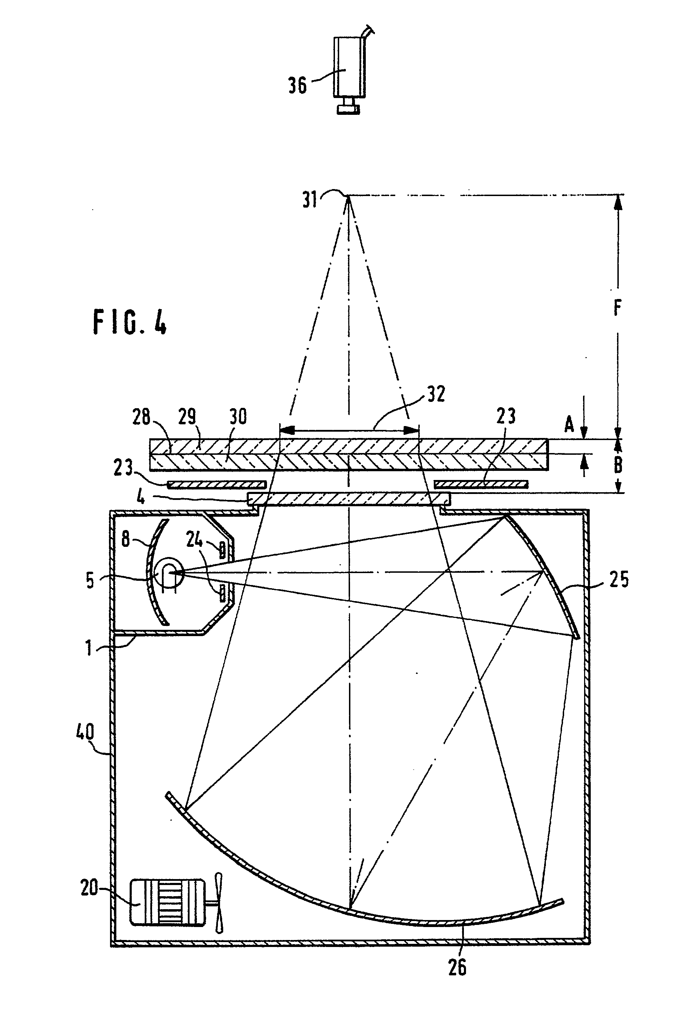

Wie Fig. 4 erkennen läßt, ist nur ein Teil der Arbeitsplatte 29 beleuchtet. Das optische System 1 kann deshalb als Ganzes relativ zur Arbeitsplatte 29 verfahrbar gemacht werden. Dieses Prinzip läßt sich extrem erweitern, indem die beleuchtete Fläche 32 der Arbeitsplatte 29 entweder als schmaler Streifen oder gar als enger, punktförmiger Fleck gewählt wird. Hierbei ist eine Bewegung zwischen optischem System 1 und Arbeitsplatte 29 bzw. dem auf der Arbeitsplatte 29 liegenden, zu untersuchenden Gewebe bzw. Körperteil unbedingt erforderlich. In diesen Fällen kann eine Auswertung praktisch nur noch mit Hilfe eines optischen und insbesondere optoelektronischen Bildaufnehmers 36 erfolgen, wobei auch eine Bildauswertung mit Hilfe eines Elektronenrechners denkbar ist.

Praktische Versuche mit der Durchleuchtungsvorrichtung der Fig. 4 haben gezeigt, daß bereits eine unterschiedliche Dicke A der Arbeitsplatte 29, an deren Unterseite ja die lichtstreuende Fläche 28 angeordnet ist, einen merkbaren Einfluß auf die Erkennbarkeit von Fremdkörpern usw. im Inneren des durchleuchteten Gewebes hat. Glücklicherweise läßt sich dieser Einfluß jedoch durch eine Justierung des Abstandes B zwischen der Arbeitsplatte 29 und dem optischen System 1 ausgleichen.

Sollen große Flächen 32 der Arbeitsplatte 29 beleuchtet werden, können Probleme mit der ausreichenden Helligkeit auftreten. Man wird dann mehrere Lampen 5 parallel schalten, wobei es bei geeigneter Anordnung der optischen Systeme - Röhrenlampen, Spiegel, Blenden, Linsen usw. - möglich ist, von jeder Lampe eine eigene Fläche 32 ausleuchten zu lassen, wobei diese Flächen 32 nahtlos aneinander stoßen.

## Patentansprüche

1. Vorrichtung zum Durchstrahlen von biologischem Gewebe mit Licht zur Sichtbarmachung von darin enthaltenen Körpern, enthaltend eine lichtdurchlässige Arbeitsplatte (2, 29) und wenigstens eine künstliche Lichtquelle (5), die unter der Arbeitsplatte (2, 29) angeordnet ist und deren Licht über, ein optisches System (1) gebündelt auf die Arbeitsplatte (2, 29) gerichtet wird, dadurch gekennzeichnet, daß das Licht so gebündelt ist, daß der virtuelle Brennpunkt (31) in einem Abstand (F) oberhalb der Oberfläche der Arbeitsplatte (2, 29) liegt, daß in einem Abstand (A) unter der Oberfläche der Arbeitsplatte (2, 29) eine lichtstreuende Fläche (28) angeordnet ist und daß die Lichtquelle (5) so angeordnet und abgeschirmt ist, daß kein direktes Licht die Arbeitsplatte (2, 29) trifft.

2. Vorrichtung nach Anspruch 1, dadurch gekennzeichnet, daß das optische System (1) aus einem länglichen, teilweise verspiegelten Gehäuse (3) mit etwa parabolischem Querschnitt besteht, dessen Oberseite mit einer Glasplatte (4) abgedeckt ist, und daß das optische System (1) in einen Lichtkasten (10) eingesetzt ist, der aus zwei Teilen (10.1, 10.2) zusammengesetzt an Führungsschienen (11) leichtgängig gelagert ist.

3. Vorrichtung nach Anspruch 1, dadurch gekennzeichnet, daß das optische System (1) in einem Gehäuse (40) gekapselt ist und daß die Öffnung, durch die der Lichtstrahl hindurch auf die Unterseite der Arbeitsplatte (29) gerichtet ist, durch eine Glasplatte (4) abgedeckt ist.

4. Vorrichtung nach Anspruch 1, 2, oder 3, dadurch gekennzeichnet, daß im Lichtweg wenigstens ein IR-durchlässiger Spiegel (7, 8, 25, 26) bzw. ein IR-Sperrfilter (4) angeordnet ist.

5. Vorrichtung nach einem der Ansprüche 1 bis 4, dadurch gekennzeichnet, daß das optische System (1) und/oder die Arbeitsplatte (2, 29) mit einem Kühlmittel forciert gekühlt sind.

6. Vorrichtung nach einem der Ansprüche 1 bis 5, dadurch gekennzeichnet, daß unterhalb der lichtstreuenden Fläche (28) verstellbare Blenden (23, 24) vorgesehen sind, um die beleuchtete Fläche (32) der Arbeitsplatte (29) zu begrenzen.

7. Vorrichtung nach einem der Ansprüche 1 bis 6, dadurch gekennzeichnet, daß die lichtstreuende Fläche (28) sich zwischen zwei schützenden Platten (29, 30), vorzugsweise aus gehärtetem Glas, befindet und vorzugsweise aus einer Schicht Diamantstaub besteht.

8. Vorrichtung nach einem der Ansprüche 1 bis 7, dadurch gekennzeichnet, daß mehrere Lichtquellen (5) vorgesehen sind, die bei Bedarf nebeneinanderliegende Flächen (32) der Arbeitsplatte (2, 29) ausleuchten.

9. Vorrichtung nach einem der Ansprüche 1 bis 8, dadurch gekennzeichnet, daß der Abstand (B) des optischen Systems (1) zur Oberfläche der Arbeitsplatte (2, 29) justierbar ist.

10. Vorrichtung nach einem der Ansprüche 1 bis 9, dadurch gekennzeichnet, daß das optische System (1) und die Arbeitsplatte (2, 29) relativ zueinander hin- und herbewegbar sind, wobei das optische System (1) die Arbeitsplatte (2, 29) nur streifenförmig oder punktförmig beleuchtet.

## Claims

1. An apparatus for irradiating biological tissue with light to render visible bodies contained therein, comprising a transparent work plate (2, 29) and at least one artificial light source (5) which is arranged under the work plate (2, 29) and whose light focused by an optical system (1), is directed on to the work plate (2, 29), characterised in that the light is focused so that the virtual focal point (31) is situated at a distance (F) above the surface of the work plate (2, 29), in that a light-diffusing surface (28) is disposed at a distance (A) below the surface of the work plate (2, 29) and in that the light source (5) is so arranged and screened that no direct light reaches the work plate (2, 29).

2. An apparatus according to Claim 1, characterised in that the optical system (1) comprises an oblong, partly mirrored housing (3) of approximately parabolic cross-section, the upper side of which is covered by a glass plate (4), and in that the optical system (1) is inserted in a light box (10) composed of two parts (10.1, 10.2) and mounted to be easily movable on guide rails (11).

3. An apparatus according to Claim 1, characterised in that the optical system (1) is enclosed in a housing (40) and that the opening through which the light beam is directed on to the underside of the work plate (29) is covered by a glass plate (4).

4. An apparatus according to Claim 1, 2 or 3, characterised in that at least one IR-permeable mirror (7, 8, 25, 26) and/or an IR barrier filter (4) is disposed in the light path.

5. An apparatus according to any one of Claims 1 to 4, characterised in that the optical system (1) and/or the work plate (2, 29) is forced-cooled by a cooling means.

6. An apparatus according to any one of Claims 1 to 5, characterised in that adjustable screens (23, 24) are provided below the light-diffusing surface (28) so as to delimit the illuminated area (32) of the work plate (29).

7. An apparatus according to any one of Claims 1 to 6, characterised in that the light-diffusing surface (28) is disposed between two protective plates (29, 30), preferably consisting of toughened glass, and is preferably composed of a layer of diamond dust.

8. An apparatus according to any one of Claims 1 to 7, characterised in that a plurality of light sources (5) are provided which, if required, illuminate adjacent areas (32) of the work plate (2, 29).

9. An apparatus according to any one of Claims 1 to 8, characterised in that the distance (B) of the optical system (1) from the surface of the work plate (2, 29) is adjustable.

10. An apparatus according to any one of Claims 1 to 9, characterised in that the optical system (1) and the work plate (2, 29) can be moved relative to one another in reciprocating motion, the optical system (1) illuminating the work plate (2, 29) only in strip-like or dot-like manner.

## Revendications

1. Dispositif pour éclairer, par transmission, des tissus biologiques par de la lumière, pour rendre visibles des corps contenus dans ce tissu, comprenant une plaque de travail transparente (2, 29) et au moins une source de lumière artificielle (5), disposée au-dessous de la plaque de travail (2, 29) et dont la lumière concentrée par un système optique (1) est dirigée sur la plaque de travail (2, 29) caractérisé en ce que la lumière est concentrée de manière que le point focal virtuel (31) soit situé à une certaine distance (F) au-dessus de la surface de la plaque de travail (2, 29), en ce qu'il est disposé, à une certaine distance (A) au-dessous de la surface de la table de travail (2, 29), une surface de dispersion de la lumière (28) et en ce que la source de lumière (5) est disposée et canalisée, par des écrans, de manière qu'aucune lumière directe ne tombe sur la plaque de travail (2, 29).

2. Dispositif selon la revendication 1, caractérisé en ce que le système optique (1) est constitué par un boîtier (3) longitudinal, partiellement équipé de miroirs de section sensiblement parabolique et dont le côté supérieur est recouvert par une plaque de verre (4), et en ce que le système optique (1) est monté dans une boîte lumineuse (10) qui est formée par l'assemblage de deux parties (10.1, 10.2) et montée de façon à pouvoir se déplacer facilement sur des rails de guidage (11).

3. Dispositif selon la revendication 1, caractérisé en ce que le système optique (1) est encapsulé dans un boîtier (40) et en ce que l'ouverture, par laquelle est dirigé le faisceau lumineux sur le côté inférieur de la plaque de travail (29), est recouverte par une plaque de verre (4).

4. Dispositif selon l'une quelconque des revendications 1 à 3, caractérisé en ce qu'un miroir transparent aux IR au moins (7, 8, 25, 26) ou un filtre d'arrêt IR (4) est monté sur le parcours suivi par la lumière.

5. Dispositif selon l'une quelconque des revendications 1 à 4, caractérisé en ce que le système optique (1) et/ou la plaque de travail (2, 29) est refroidi de façon forcée par un agent de refroidissement.

6. Dispositif selon l'une quelconque des revendications 1 à 5, caractérisé en ce que sont prévus, au-dessous de la surface de dispersion de la lumière (28) des écrans réglables (23, 24) de manière à limiter la surface éclairée (32) de la plaque de travail (29).

7. Dispositif selon l'une quelconque des revendications 1 à 6, caractérisé en ce que la surface de dispersion de la lumière (28) se trouve prise en sandwich entre deux plaques de protection (29, 30), de préférence en verre durci, et est constituée, de préférence, par une couche de poussière de diamant.

8. Dispositif selon l'une quelconque des revendications 1 à 7, caractérisé en ce que sont prévues plusieurs sources de lumière (5) qui éclairent, en cas de besoin, des surfaces voisines les unes des autres (32) de la plaque de travail (2, 29).

9. Dispositif selon l'une quelconque des revendications 1 à 8, caractérisé en ce que la distance (B) séparant le système optique (1) de la surface de la plaque de travail (2, 29) peut être ajustée.

10. Dispositif selon l'une quelconque des revendications 1 à 9, caractérisé en ce que le système optique (1) et la plaque de travail (2, 29) peuvent être déplacés en va-et-vient l'un par rapport à l'autre, le système optique (1) n'éclairant la plaque de travail (2; 29) que sous forme d'une bande ou d'un point.

FIG.1

FIG.2

FIG.3

FIG. 4